# EUROPEAN PATENT APPLICATION

(11) **EP 4 141 106 A1**
(43) Date of publication of application: **01.03.2023**
(21) Application number: 22780870.6
(22) Date of filing: 29.03.2022
(51) Int. Cl.: C12N 5/02, C12N 5/071, C12N 5/0775

(54) **METHOD FOR PRODUCING CULTURE AND CELL COLLECTION METHOD**

(30) Priority: 31.03.2021 JP 2021060494
(71) Applicant: Showa Denko Materials Co., Ltd., Tokyo 100-6606 (JP)
(72) Inventor: SATO, Yushi, Tokyo 100-6606 (JP); OKANOJO, Masahiro, Tokyo 100-6606 (JP); CHEN, Shangwu, Tokyo 100-6606 (JP); TAKAHASHI, Ryosuke, Tokyo 100-6606 (JP)
(74) Representative: Winter, Brandl - Partnerschaft mbB
(86) International application number: PCT/JP2022/015315
(87) International publication number: WO 2022/210659

(57) **Abstract**

A culture manufacturing method that includes: bringing adherent cells into contact with a dissolvable culture carrier that is larger than the size of the adherent cells and disposing the adherent cells on the surface of the dissolvable culture carrier, subjecting the adherent cells disposed on the surface of the dissolvable culture carrier to suspension culture in a culture medium, subjecting the dissolvable culture carrier to a modification treatment that modifies at least a portion of the surface in order to detach the adherent cells in the suspension culture from the surface of the dissolvable culture carrier, and, following the modification treatment, separating and harvesting the adherent cells from the modified dissolvable culture carrier that is larger than the size of the adherent cells on the basis of the size difference.

## Description

### TECHNICAL FIELD

The disclosure relates to a culture manufacturing method, a cell harvest method, a kit, and a cell-containing composition.

### BACKGROUND ART

Cell culture can be broadly classified into plate culture in which adherent cells are grown in a single layer on a plate-like culture carrier, and suspension culture in which adherent cells are adhered to the surface of a mainly particulate culture carrier and cell culturing is conducted in a state where a complex of the adherent cells and the culture carrier is suspended in the culture system.

In suspension culture, the culture carrier causes efficient cell growth by functioning as a scaffold for cell proliferation, and following the cell culture, cell harvest is conducted by detaching the cells from the culture carrier using an enzyme or chelating agent or the like. Culture carriers that can be used in suspension culture include both dissolvable carriers and indissolvable carriers, and in those cases where a dissolvable culture carrier is used, the culture carrier is dissolved following cell culture, enabling the lone cells to be harvested with a filter or the like. In large-scale culturing of cells, this type of suspension culture can be used favorably. In the case of a dissolvable culture carrier, a dissolution protocol appropriate for the constituent components of the carrier is established, and following cell culture, the carrier can be favorably dissolved in accordance with that protocol.

For example, JP 2016-523086 A discloses a cell culture article including a substrate containing a polygalacturonic acid compound such as pectic acid and an adhesive polymer on the substrate surface, and proposes that the culture carrier can be dissolved by using a pectinase and a chelating agent, enabling easy collection of the cells.

### SUMMARY OF INVENTION

### PROBLEMS INVENTION AIMS TO SOLVE

In those cases where cells obtained in a large amount using a culture carrier are, for example, used as a cell preparation, it is desirable to remove, as far as possible, any impurities other than the cells. However, when a dissolvable culture carrier is dissolved favorably in accordance with the established protocol, dissolution residues are generated within the cell suspension. Because many of these dissolution residues are either of a similar size to the cells, or smaller than the cells, even if an attempt is made to harvest only the cells from the cell suspension, completely avoiding contamination by dissolution residues is difficult.

The disclosure provides a culture manufacturing method and a cell harvest method that suffer little impurity contamination and yield high adherent cell purity, as well as kits that are suited to these methods and a cell-containing composition having high adherent cell purity.

### MEANS FOR SOLUTION OF THE PROBLEMS

Embodiments of the disclosure are as follows.
[1] A culture manufacturing method that includes: bringing adherent cells into contact with a dissolvable culture carrier that is larger than the size of the adherent cells and disposing the adherent cells on the surface of the dissolvable culture carrier, subjecting the adherent cells disposed on the surface of the dissolvable culture carrier to suspension culture in a culture medium, subjecting the dissolvable culture carrier to a modification treatment that modifies at least a portion of the surface in order to detach the adherent cells in the suspension culture from the surface of the dissolvable culture carrier, and, following the modification treatment, separating and harvesting the adherent cells from the modified dissolvable culture carrier that is larger than the size of the adherent cells on the basis of the size difference.
[2] The culture manufacturing method according to [1], wherein the dissolvable culture carrier contains a polysaccharide, a protein, or a combination thereof.
[3] The culture manufacturing method according to [1] or [2], wherein the dissolvable culture carrier contains at least one substance selected from the group consisting of dextran, cellulose, collagen, gelatin, polygalacturonic acid, alginic acid, and derivatives thereof.
[4] The culture manufacturing method according to any one of [1] to [3], wherein the dissolvable culture carrier is porous.
[5] The culture manufacturing method according to any one of [1] to [4], wherein the modification treatment includes modifying the dissolvable culture carrier using a surface modifier.
[6] The culture manufacturing method according to any one of [1] to [5], wherein the adherent cells and the modified dissolvable culture carrier are separated using a separation device.
[7] The culture manufacturing method according to [6], wherein the separation device has a pore size of 20 to 50 µm.
[8] The culture manufacturing method according to any one of [1] to [7], wherein the size of the modified dissolvable culture carrier has a minimum diameter that exceeds 50 µm.
[9] A cell harvest method that includes: providing a cell suspension containing a cell complex which contains adherent cells and a dissolvable culture carrier that is larger than the size of the adherent cells, and has the adherent cells disposed on the surface of the dissolvable culture carrier, subjecting the cell complex in the cell suspension to a modification treatment that modifies at least a portion of the surface of the dissolvable culture carrier in order to detach the adherent cells from the surface of the dissolvable culture carrier, and separating and harvesting the adherent cells in the cell suspension from the modified dissolvable culture carrier that is larger than the size of the adherent cells on the basis of the size difference.
[10] A kit including a dissolvable cell culture carrier, and instructions that disclose use of the dissolvable cell culture carrier in the culture manufacturing method according to any one of [1] to [8] or the cell harvest method according to [9].
[11] A kit including a surface modifier for a modification treatment of a dissolvable cell culture carrier, and instructions that disclose use of the surface modifier in the culture manufacturing method according to any one of [1] to [8] or the cell harvest method according to [9].
[12] A cell-containing composition containing adherent cells, wherein the amount of dissolvable culture carrier dissolution residues having a size of 20 µm or smaller is 30 particles or less per 1 × 10⁴ cells.
[13] Use of a dissolvable cell culture carrier in the culture manufacturing method according to any one of [1] to [8], the cell harvest method according to [9], or the kit according to [10].
[14] Use of a surface modifier for a modification treatment of a dissolvable cell culture carrier in the culture manufacturing method according to any one of [1] to [8], the cell harvest method according to [9], or the kit according to [10].

### EFFECTS OF THE INVENTION

The disclosure is able to provide a culture manufacturing method and a cell harvest method that suffer little impurity contamination and yield high adherent cell purity, as well as kits that are suited to these methods and a cell-containing composition having high adherent cell purity.

### BRIEF DESCRIPTION OF THE DRAWINGS

FIG. 1 is a series of single visual field phase contrast microscope photographs following the modification treatments of cell suspensions manufactured in the examples and comparative examples.
FIG. 2 is a single visual field phase contrast microscope photograph following the modification treatment and pipetting of a cell suspension manufactured in a comparative example.

### EMBODIMENTS FOR CARRYING OUT THE INVENTION

In this description, numerical ranges indicated using the expression "a to b" indicate a range that includes the numerical values before and after the "to" as the minimum value and maximum value respectively. In the case of numerical ranges listed in a stepwise manner in this description, the upper limit value or lower limit value from the numerical range of any particular step may be arbitrarily combined with the upper limit value or lower limit value from the numerical range of another step. In a numerical range disclosed in this description, the upper limit value or lower limit value from the numerical range may be used to replace a value shown in an example. In this description, unless specifically stated otherwise, the amount of each component in a composition, in the case where a plurality of substances corresponding with any particular component exist in the composition, means the total amount of the plurality of substances that exist in the composition. In this description, the term "step" refers to not only independent steps, but also includes steps that achieve an intended action, even if the step cannot be clearly differentiated from another step. Unless state otherwise, elements indicate either a single element or a plurality of elements.

Embodiments of the disclosure are described below. The disclosure is not limited by the examples in the following embodiments. Terms and expressions used in the following description are not limited by the specific examples presented in the examples described below.

### [Culture Manufacturing Method]

A culture manufacturing method according to one embodiment of the disclosure includes: bringing adherent cells into contact with a dissolvable culture carrier that is larger than the size of the adherent cells and disposing the adherent cells on the surface of the dissolvable culture carrier, subjecting the adherent cells disposed on the surface of the dissolvable culture carrier to suspension culture in a culture medium, subjecting the dissolvable culture carrier to a modification treatment that modifies at least a portion of the surface in order to detach the adherent cells in the suspension culture from the surface of the dissolvable culture carrier, and, following the modification treatment, separating and harvesting the adherent cells from the modified dissolvable culture carrier that is larger than the size of the adherent cells on the basis of the size difference.

According to this culture manufacturing method, a culture having little impurity contamination and high adherent cell purity can be obtained. In other words, in this culture manufacturing method, in order to detach the adherent cells from the dissolvable culture carrier having the adherent cells on the surface thereof, rather than dissolving the entire dissolvable culture carrier, a modification treatment is conducted that modifies at least a portion of the surface. The adherent cells employ the surface of the dissolvable culture carrier as an adhesion scaffold, and it is thought that the modification treatment causes a loss in this adhesion scaffold, resulting in cell detachment from the culture carrier, although the disclosure is not limited to this theory. Further, because the modification treatment stops at the modification of the surface of the dissolvable culture carrier, the large amounts of dissolution residues that develop when the entire culture carrier is dissolved do not occur, and the size of the modified dissolvable culture carrier obtained following the modification treatment does not reduce to a size smaller than the size of the adherent cells. As a result, based on the size difference between the adherent cells and the modified dissolvable culture carrier, the adherent cells can be separated and harvested from the modified dissolvable culture carrier simply and with good precision.

It is preferable that the obtained culture contains the adherent cells but does not contain the dissolvable culture carrier, and that the amount of residues derived from the dissolvable culture carrier is extremely low. More specifically, in the obtained culture, it is preferable that the amount of residues such as dissolution residues generated when the dissolvable culture carrier is dissolved and having a size similar to or smaller than the size of the cells is extremely low.

In the following description, a cell complex means a complex containing adherent cells and a dissolvable culture carrier, with the adherent cells disposed on the surface of the dissolvable culture carrier. A cell suspension means a liquid containing adherent cells.

The culture manufacturing method may include bringing adherent cells into contact with a dissolvable culture carrier that is larger than the size of the adherent cells, and disposing the adherent cells on the surface of the dissolvable culture carrier.

The adherent cells may be cells that can be supported on the surface of the dissolvable culture carrier, and for which cell growth can be accelerated with the cells disposed on the surface of the dissolvable culture carrier. The adherent cells may constitute a cell suspension in combination with an aqueous medium. Examples of aqueous media that may constitute the cell suspension include culture media, physiological saline solution, and phosphate buffers and the like.

The cells are preferably animal-derived cells, and are more preferably mammalian-derived cells. Examples of the mammals include humans, monkeys, chimpanzees, cows, pigs, horses, sheep, goats, rabbits, rats, mice, marmots, dogs and cats. There are no particular limitations on the tissue from which the cells are derived, and examples include the skin, liver, kidneys, muscles, bone, blood vessels, blood, or nerve tissue. The cells may be primary culture cells, cultured cell lines, or recombinant culture cell lines or the like.

Examples of stem cells include somatic stem cells such as mesenchymal stem cells, hematopoietic stem cells, neural stem cells, myeloid stem cells, germline stem cells and dental pulp stem cells, and mesenchymal stem cells are preferred. The term "mesenchymal stem cells" refers broadly to somatic stem cells which exist in various tissues in the body, and can be differentiated into all or some types of mesenchymal cells such as osteoblasts, chondrocytes and adipocytes. Examples of mesenchymal stem cells include bone marrow-derived mesenchymal stem cells, umbilical cord-derived mesenchymal stem cells, and adipose tissue-derived mesenchymal stem cells.

Pluripotent stem cells such as induced pluripotent stem cells (iPS cells), embryonic stem cells (ES cells), embryonic germ cells (EG cells), multipotent germ stem cells (mGS cells), embryonic carcinoma cells (EC cells) and Muse cells may be used as the stem cells.

The cells may be differentiated cells such as endothelial cells, epidermal cells, epithelial cells, myocardial cells, myoblasts, nerve cells, bone cells, osteoblasts, fibroblasts, adipose cells, hepatic cells, renal cells, pancreatic cells, adrenal gland cells, periodontal ligament cells, gingival cells, periosteal cells, skin cells, dendritic cells, macrophages and lymphocyte cells; or precursor cells that represent a preliminary stage between stem cells and these differentiated cells.

A single type of the above cells may be used alone, or combination of two or more types of cell may be used.

The dissolvable culture carrier describes a culture carrier formed from a material that can be decomposed using an enzyme or heat or the like. The dissolvable culture carrier can support adherent cells on the carrier surface, and can form a complex containing the dissolvable culture carrier and adherent cells in which the adherent cells are disposed on the carrier surface. Further, the dissolvable culture carrier is preferably a substance for which the surface can be modified by a modification treatment, causing a weakening of the action that supports the adherent cells.

There are no particular limitations on the dissolvable culture carriers that can be used, provided the carrier can be used as a scaffold for proliferation of the adherent cells on the carrier surface. For example, the dissolvable culture carrier may contain a polysaccharide, a protein, a polypeptide, or a combination of these substances, preferably contains a polysaccharide, a protein, or a combination thereof, and more preferably contains a polysaccharide. The dissolvable culture carrier may be either a natural polymer or a synthetic polymer.

Examples of the polysaccharides include polygalacturonic acids such as pectin and pectic acid; as well as alginic acid, cellulose, dextran, agarose, chitosan, glycosaminoglycan, and derivatives of these substances. These polysaccharides may also have a crosslinked structure, such as, crosslinked cellulose, crosslinked dextran, crosslinked agarose and crosslinked chitosan. Among these, polygalacturonic acids, polygalacturonic acid esters, alginic acid, alginic acid esters, cellulose, crosslinked cellulose, dextran, crosslinked dextran, and combinations of these substances are preferred.

Examples of the proteins include collagen and gelatin and the like.

The dissolvable culture carrier preferably contains at least one substance selected from the group consisting of dextran, cellulose, collagen, gelatin, polygalacturonic acid, alginic acid, and derivatives of these substances. The cellulose and dextran may be either crosslinked or not crosslinked. Among the various possibilities, the dissolvable culture carrier preferably contains dextran, crosslinked dextran, or a combination thereof, and more preferably contains crosslinked dextran.

In order to improve the support properties (adhesion) of the adherent cells, cationic functional groups may be introduced onto the surface of the dissolvable culture carrier. Examples of the cationic functional groups include amino groups having a substituent, primary amino groups, and quaternary ammonium groups. Examples of the amino groups having a substituent include amino groups having an alkyl group of 1 to 10 carbon atoms, and preferably 1 to 4 carbon atoms, and either monoalkylamino groups or dialkylamino groups may be used, with specific examples including a methylamino group, dimethylamino group, ethylamino group, diethylamino group, methylethylamino group, propylamino group, dipropylamino group, butylamino group and dibutylamino group. The cationic functional groups may also be functional groups having a substituted or unsubstituted amino group, such as aminoalkyl groups, and specific examples include an aminoethyl group, aminopropyl group, methylaminoethyl group, dimethylaminoethyl group, ethylaminoethyl group, and diethylaminoethyl group. One of these types of cationic functional groups may be introduced alone onto the surface of the dissolvable culture carrier, or a combination of two or more types may be introduced.

Further, from the viewpoint of promoting the adherent cell support properties, a cell-adhesive polymer may be disposed at the surface of the dissolvable culture carrier, and may constitute part or all of the surface of the dissolvable culture carrier. Examples of the cell-adhesive polymer include collagen, gelatin, alginic acid and salts thereof, Matrigel (a registered trademark) (BD Biosciences Ltd.), hyaluronic acid, laminin, fibronectin, vitronectin, elastin, heparan sulfate, dextran, dextran sulfate and chondroitin sulfate. Among these, cell-adhesive polypeptides such as collagen, gelatin, laminin, fibronectin and vitronectin are preferred.

Specific examples of the dissolvable culture carrier include dextran or crosslinked dextran having dialkylamino groups, and dextran or crosslinked dextran that has been coated with collagen or modified collagen.

The shape of the dissolvable culture carrier is not particularly limited, provided the shape is capable of supporting the adherent cells, and examples include spherical shapes, flat shapes, cylindrical shapes, plate shapes and prismatic shapes. The dissolvable culture carrier preferably includes a spherical dissolvable culture carrier

The dissolvable culture carrier may be a porous culture carrier having pores within the surface, the interior, or both the surface and the interior, or may be a solid culture carrier having no pores within the interior, or having no pores within either the surface or the interior. By using a porous culture carrier as the dissolvable culture carrier, the surface area of the dissolvable culture carrier can be increased, enabling an increase in the number of adherent cells supported on the surface of the dissolvable culture carrier.

Examples of the dissolvable culture carrier according to one embodiment of the disclosure include the Cytodex (a registered trademark, hereafter omitted) series, Cytopore series and Sephadex (a registered trademark, hereafter omitted) series available from Cytiva Co., Ltd.

The dissolvable culture carrier may have a size that is larger than the adherent cells. Confirmation that the size of the dissolvable culture carrier is larger than the adherent cells can be made, for example, by passing the cell suspension containing the adherent cells and the dissolvable culture carrier though a filter having a prescribed pore size. The pore size of filters that may be used for this confirmation may be any size within the range larger than the size of the adherent cells but smaller than the size of the dissolvable culture carrier.

More specifically, although the size of the adherent cells varies depending on the cell variety, the size may be within a range from 1 to 50 µm, from 10 to 30 µm, from 10 to 20 µm, or from 10 to 18 µm. Further, the adherent cells may be of a size that enables passage through a filter having a pore size that is, for example, 10 µm or larger, but not more than 50 µm, not more than 30 µm, not more than 20 µm, or 18 µm or smaller.

Prior to the modification treatment, the average particle size (D50) of the dissolvable culture carrier is, for example, within a range from 40 to 1,000 µm, and may be from 50 to 500 µm, from 100 to 250 µm, or from 100 to 200 µm. Within this range, the size of the dissolvable culture carrier prior to the modification treatment is larger than the size of the adherent cells, can support a plurality of adherent cells on the surface of each dissolvable culture carrier, and can contribute to an acceleration of cell proliferation. Further, within this range, because the size of the dissolvable culture carrier prior to the modification treatment is larger than the size of the adherent cells, maintaining a state in which the size of the dissolvable culture carrier after the modification treatment is still larger than the size of the adherent cells is easier, meaning the adherent cells can be easily separated from the modified dissolvable culture carrier based on the cell size.

In this disclosure, the average particle size of the dissolvable culture carrier is the value measured for the volume-based median size (D50) in a physiological saline solution or in a culture medium. The average particle size of the dissolvable culture carrier can be measured using a laser diffraction and scattering-type particle size distribution analyzer. However, in those cases where the dissolvable culture carrier is a commercially available product, reference may simply be made to the product information included with the product.

Prior to the modification treatment, the size of the dissolvable culture carrier preferably has a minimum diameter that exceeds 50 µm. Prior to the modification treatment, the size of the dissolvable culture carrier has a minimum diameter which may, for example, exceed 50 µm, or be at least 80 µm, at least 100 µm, at least 150 µm, or 170 µm or larger. By ensuring that the size of the dissolvable culture carrier prior to the modification treatment falls within this range, the size difference between the modified dissolvable culture carrier following modification treatment and the adherent cells can be more easily maintained, and as a result, the adherent cells can be more easily separated and harvested from the modified dissolvable culture carrier.

In this disclosure, the minimum diameter of the dissolvable culture carrier means either the value obtained using the measurement method described below or the D5 value. The minimum diameter of the dissolvable culture carrier can be confirmed by conducting phase contrast microscope observations at a plurality of locations, for example 5 visual fields, and referring to a measure or scale bar attached to the microscope. In such cases, the dissolvable culture carrier may be subjected to fluorescent coloration. Further, the minimum diameter of the dissolvable culture carrier may also be confirmed easily using a filter having a pore size of 50 µm. In this disclosure, the D5 value for the dissolvable culture carrier refers to the value obtained by measuring the volume-based particle size distribution in either a physiological saline solution or in a culture medium, and represents the particle size at a cumulative volume of 5% from the small particle size side of the distribution. The particle size distribution of the dissolvable culture carrier can be measured using a laser diffraction and scattering-type particle size distribution analyzer. However, in those cases where the dissolvable culture carrier is a commercially available product, reference may simply be made to the product information included with the product.

The average particle size (D50), the D5 value, and the minimum diameter for the dissolvable culture carrier before and after the modification treatment can be measured using the same procedures.

Examples of the method used for bringing the adherent cells and the dissolvable culture carrier into contact include introducing the dissolvable culture carrier and the adherent cells into a culture medium or the like inside a cell culture container, prior to starting the culture, at the time of starting the culture, or after starting the culture. There are no particular limitations on the order of introduction. There are no particular limitations on the amounts of the dissolvable culture carrier and the adherent cells introduced into the medium, and these amounts may be selected appropriately in accordance with the scale of the cell culture and the equipment and the like.

The culture manufacturing method may include subjecting the adherent cells disposed on the surface of the dissolvable culture carrier to suspension culture within a culture medium.

The adherent cells disposed on the surface of the dissolvable culture carrier may simply be used in the above form obtained by bringing the adherent cells and the dissolvable culture carrier into contact within a culture medium. In other words, in those cases where the adherent cells and the dissolvable culture carrier are introduced into a culture medium, suspension culture may be started from that state, or a culture medium may be mixed with a liquid obtained by bringing the adherent cells and the dissolvable culture carrier into contact within a medium other than a culture medium, such as a buffer solution, and a suspension culture then started.

Inside the culture container, as proliferation of the adherent cells proceeds with the progression of the suspension culture, a cell complex composed of a complex of the adherent cells and the dissolvable culture carrier is formed, and the adherent cells grow on the dissolvable culture carrier of this cell complex. The cell suspension may contain not only this cell complex, but also cell clusters (aggregates) formed by the aggregation of a plurality of cells, and culture carriers to which no cells are adhered. Examples of culture carriers to which no cells are adhered include additional dissolvable culture carrier that has been introduced during the suspension culture.

The seeding density of the adherent cells at the start of the culture varies depending on the cell type and the culture conditions and the like, and may be, for example, within a range from 5×10² to 2×10⁵ cells/mL, from 1×10³ to 1×10⁵ cells/mL, or from 1×10³ to 5×10⁴ cells/mL. The concentration of the dissolvable culture carrier in the cell suspension at the start of the culture may be set, for example, to a value within a range from 0.05 to 50 g/L, from 0.1 to 10 g/L, or from 0.3 to 5 g/L. In those cases where the dissolvable culture carrier is porous, the concentration of the dissolvable culture carrier may be adjusted so that the surface area of the dissolvable culture carrier falls within a range from 0.1 to 30 cm²/mL, from 0.5 to 20 cm²/mL, from 1 to 10 cm²/mL, or from 3 to 8 cm²/mL. The adherent cells and the dissolvable culture carrier may be used in appropriate combinations within the above ranges.

The culture medium used for culturing the adherent cells preferably contains an inorganic salt, an amino acid, sugar and water. The culture medium may also contain optional components such as serum, vitamins, hormones, antibiotics, growth factors and adhesion factors. Culture media that are known as basal media for cell culture may be used as the culture medium. In other words, any culture medium that is known for use in culturing the selected cells may be used as the medium without any particular limitations.

Examples of the culture medium include, but are not limited to, DMEM (Dulbecco's Modified Eagle's Medium), MEM (Eagle's Minimum Essential Medium), αMEM medium (α-Modified Eagle's Minimum Essential Medium), GMEM (Glasgow Minimum Essential Medium), IMDM (Iscove's Modified Dulbecco's Medium), Ham's F12 (Ham's Nutrient Mixture F12), RPMI-1640 (RPMI-1640 medium), McCoy's 5A (McCoy's 5A Medium), MSC growth medium 2 (PromoCell GmbH), Prime XV XSFM (Irvine Scientific, Inc.). One of these culture media may be used alone, or a combination of two or more media may be used.

In those cases where a serum is added to the culture medium, serums such as fetal bovine serum (FBS), horse serum and human serum may be used.

The culture medium used for the culture may be free of xenogeneic components. Culture media that are free of xenogeneic components may include serum substitute additives (for example, Knockout Serum Replacement (KSR) (Invitrogen Corporation), Chemically-defined Lipid Concentrate (Gibco Inc.), and Glutamax (Gibco Inc.) and the like) instead of animal-derived serum.

In addition, serum-free culture media such as Essential 8 (Thermo Fisher Scientific Inc.), mTeSR1 (STEMCELL Technologies Inc.), the StemFit series (Takara Bio Inc.) and StemFlex (Thermo Fisher Scientific Inc.) may also be used.

Additives may be added to the culture medium if necessary. Examples of these additives include vitamins such as vitamin A, vitamin B 1, vitamin B2, vitamin B6, vitamin B 12, vitamin C and vitamin D; coenzymes such as folic acid; amino acids such as glycine, alanine, arginine, asparagine, glutamine, isoleucine and leucine; sugars and organic acids that act as carbon sources such as lactic acid; growth factors such as EGF, FGF, PFGF and TGF-β; interleukins such as IL-1 and IL-6; cytokines such as TNF-α, TNF-β and leptin; metal transporters such as transferrin; metal ions such as iron ions, selenium ions and zinc ions; SH reagents such as β-mercaptoethanol and glutathione; and proteins such as albumin.

There are no particular limitations on the cell culture method, and a method suited to the particular cell may be used. The temperature of the cell culture, expressed as the temperature of the cell suspension, may be, for example, within a range from 20 to 45°C, or within a range from 30 to 40°C, and is preferably from 36 to 37°C. The pH of the cell culture, expressed as the pH of the cell suspension, may be, for example, within a range from 6.2 to 7.7, and is preferably a pH of 7.4. The CO₂ concentration of the cell culture, expressed as the CO₂ concentration of the cell suspension, may be, for example, within a range from 1 to 20% by volume, or within a range from 4 to 10% by volume, and is preferably from 5 to 7% by volume. In the case of a mammalian-derived cell culture, conditions including a temperature of 37°C and a carbon dioxide concentration of 5% (v/v) are typically used.

The suspension culture may be either a dynamic suspension culture in which agitation such as stirring or shaking is conducted either continuously or intermittently, or a static suspension culture in which no agitation is conducted. There are no particular limitations on the type of culture container suitable for agitation, and examples include flasks, bioreactors, tanks, and culture bags. There are no particular limitations on the method of agitation, and a method appropriate for the selected culture container may be applied. Examples of the agitation method include stirring, shaking, tilting, or a combination of these methods.

During the suspension culture, if necessary, an appropriate replacement of the culture medium may be conducted. The culture medium replacement may involve replacement of the entire medium, or replacement of a portion, such as half, of the medium. The suspension culture may be scaled up by appropriately adding more culture medium and increasing the volume of the cell suspension in accordance with the proliferation state of the adherent cells. One example of a scaling up of the culture involves a mode in which the volume is increased sequentially, for example to at least 0.3 L, at least 2 L, at least 4 L, and then 10 L or greater, but the disclosure is not limited to this mode.

The culture manufacturing method may include subjecting the dissolvable culture carrier to a modification treatment that modifies at least a portion of the carrier surface in order to enable the adherent cells in the suspension culture to be detached from the surface of the dissolvable culture carrier.

In the modification treatment, either at least a portion of the surface of the dissolvable culture carrier having the adherent cells on the surface thereof may be modified, or the entire surface of the dissolvable culture carrier may be modified. As a result, the ability of the dissolvable culture carrier to support the adherent cells weakens, and the adherent cells become able to detach from the surface of the culture carrier.

The modification treatment may be any treatment that alters the properties of the surface of the dissolvable culture carrier, for example by degrading the function of the carrier as a scaffold for cells, thus enabling detachment of the adherent cells, and the modification treatment may be selected appropriately in accordance with factors such as the type, size, and shape and the like of the dissolvable culture carrier. The modification treatment may be conducted by temperature variation or the like, or may employ a surface modifier.

In those cases where the dissolvable culture carrier is formed from a temperature-sensitive material, the adherent cells can be detached by adjusting the temperature sufficiently to alter the properties of the carrier as a cell scaffold. The adjustment of the temperature may be determined appropriately in accordance with the material used in forming the dissolvable culture carrier.

The surface modifier may be a component that is capable of modifying the properties of the surface of the dissolvable culture carrier that enable it to function as a scaffold for the cells. By using this type of surface modifier, for example, the surface of the dissolvable culture carrier is modified sufficiently to weaken the functionality of the surface of the dissolvable culture carrier as a scaffold, without causing a significant reduction in the size of the dissolvable culture carrier. As a result, the adherent cells can be more easily detached from the surface of the dissolvable culture carrier.

The size of the dissolvable culture carrier following this modification treatment is larger than the size of the adherent cells, and from the viewpoint of enabling separation using a separation device such as a filter or the like, may be at least 1.5 times, at least 2 times, at least 3 time, at least 5 times, at least 8 times, or 10 times or more, the size of the adherent cells. The size of the dissolvable culture carrier following this modification treatment, compared with the size of the dissolvable culture carrier prior to the modification treatment, may be, for example, at least 50%, at least 80%, or 90% or greater. One example of a confirmation method used for comparing the sizes of the dissolvable culture carrier before and after the modification treatment is described below. Following the modification treatment, an observation or image capture is conducted with a phase contrast microscope, and the size of the modified dissolvable culture carrier is confirmed using the measure or scale bar attached to the microscope. The average for the minimum diameter of 30 to 40 modified dissolvable culture carriers is determined within a single visual field, this operation is performed for 5 visual fields, and the average is then calculated and expressed as a percentage relative to a value of 100% for the minimum diameter of the dissolvable culture carrier prior to the modification treatment.

The average particle size (D50) of the modified dissolvable culture carrier may be, for example, within a range from 40 to 1,000 µm, and may be from 50 to 500 µm, from 100 to 250 µm, or from 100 to 200 µm. Within this range, the adherent cells can be more easily separated from the modified dissolvable culture carrier on the basis of the cell size.

The size of the modified dissolvable culture carrier preferably includes a minimum diameter exceeding 50 µm. The size of the modified dissolvable culture carrier has a minimum diameter which may, for example, exceed 50 µm, or be at least 80 µm, at least 100 µm, at least 150 µm, or 170 µm or larger. By ensuring that the size of the modified dissolvable culture carrier includes a minimum diameter that falls within this range, the size difference from the adherent cells in the subsequent separation and harvest step is sufficient to enable satisfactory separation and harvest, meaning the separation and harvest can be conducted more easily.

The average particle size (D50), the D5 value, and the minimum diameter for the modified dissolvable culture carrier can be measured using the same procedures as those described above for measurement of the dissolvable culture carrier prior to the modification treatment. In the measurements of the modified dissolvable culture carrier, a sample may be used in which the cells have already undergone separation and harvest, and have been removed from the modified cell suspension.

The conditions employed when modification treatment of the surface of the dissolvable culture carrier is conducted using a surface modifier vary depending on the type of surface modifier used and the state of the cells, but a person skilled in the art will be able to set these conditions appropriately, ensuring that the concentration and the treatment time and the like are more moderate than the conditions required for complete dissolution of the dissolvable culture carrier.

The surface modifier may be selected from among known solubilizers and decomposition agents in accordance with the type of dissolvable culture carrier being used. Specific examples of the surface modifier include enzymes, chelating agents, acids, and alkalis and the like. Examples of the enzymes include saccharide-degrading enzymes and proteases. Examples of the saccharide-degrading enzymes include dextranase, pectinase, polygalacturonase, cellulase, alginate lyase, agarase and chitosanase. Examples of the proteases include collagenase and trypsin. Examples of the chelating agents include ethylenediaminetetraacetic acid (EDTA) and ethylenediamine-N,N'-disuccinic acid (EDDS).

For example, in those cases where the dissolvable culture carrier incorporates an ester compound, the modification treatment may be conducted by hydrolyzing the surface of the dissolvable culture carrier using an acid or an alkali or the like as a catalyst. Conventional compounds may be used as the acid or alkali used in this particular application.

One of the surface modifiers described above may be used alone, or a combination of two or more surface modifiers may be used.

The surface modifier may be selected appropriately in accordance with the type of dissolvable culture carrier being used. Examples include combinations of a dissolvable culture carrier containing dextran or crosslinked dextran with a dextranase, combinations of a dissolvable culture carrier containing cellulose or crosslinked cellulose with a cellulase, combinations of a dissolvable culture carrier containing a polygalacturonic acid such as pectin with a pectinase or polygalacturonase, combinations of a dissolvable culture carrier containing collagen with a collagenase or trypsin, combinations of a dissolvable culture carrier containing gelatin with trypsin, combinations of a dissolvable culture carrier containing alginic acid with alginate lyase, and combinations of a dissolvable culture carrier having a cell-adhesive polypeptide on the surface with trypsin. The hydrolysis of ester compounds such as polygalacturonic acid esters and alginic acid esters can be accelerated by using an acid or alkali as a catalyst, thereby modifying the surface of the carrier, and therefore acids or alkalis may also be used as surface modifiers.

Conditions for the modification treatment such as the type of surface modifier, the concentration of the surface modifier, the modification treatment temperature and the modification treatment time and the like may be adjusted to ensure that the size of the dissolvable culture carrier following the modification treatment is larger than size of the cells. Examples include ensuring that the concentration of the surface modifier in the cell suspension is lower than that required for complete dissolution, ensuring that the modification treatment time is shorter than the time required for complete dissolution, ensuring that the modification treatment temperature is lower or higher than the temperature required for complete dissolution, or a combination of these conditions. By ensuring that the modification treatment with a surface modifier is conducted under conditions that are more moderate than the conditions required for complete dissolution of the dissolvable culture carrier, the modification treatment can be conducted without imparting an excessive load on the adherent cells.

Examples of other surface modifiers include pH modifiers in those cases where the dissolvable culture carrier can be subjected to surface modification by altering the pH. An acid or alkali may be used as the pH modifier. In those cases where the modification treatment is conducted using a pH modifier, the treatment may be conducted under conditions that are more moderate than the conditions required for complete dissolution of the dissolvable culture carrier.

A cell detachment treatment for promoting the detachment of the adherent cells from the surface of the dissolvable culture carrier may be conducted consecutively with, or separately from, the modification treatment. The cell detachment agent used in the cell detachment treatment may employ any conventional cell detachment agent without any particular limitations, and examples include proteases and chelating agents, with specific examples including trypsin, EDTA (ethylenediaminetetraacetic acid), and combinations thereof such as TrypLE (Thermo Fisher Scientific Inc.).

Following the modification treatment and prior to the separation and harvest of the cells, the cell suspension may be subjected to pipetting. Pipetting can be used to disperse the dissolvable culture carrier and the adherent cells detached from the dissolvable culture carrier, enabling the cells to be more easily separated and harvested in a subsequent step.

The culture manufacturing method may include a step, following the modification treatment, of separating and harvesting the adherent cells from the modified dissolvable culture carrier having a larger size than the adherent cells on the basis of the size difference.

Because the separation and harvest of the adherent cells is conducted on the basis of the size difference between the adherent cells and the modified dissolvable culture carrier, the separation and harvest of the adherent cells can be conducted simply and precisely.

As described above, because the modification treatment modifies the surface of the dissolvable culture carrier but does not completely dissolve the dissolvable culture carrier, there is a strong tendency for the carrier size to be maintained within the range described above following the modification treatment. As a result, the modified culture carrier is able to retain a size that is larger than the cells.

Because the size of the modified dissolvable culture carrier is larger than the size of the adherent cells, the adherent cells can be separated and harvested from the modified dissolvable culture carrier on the basis of the size difference. For example, a separation device such as a filter can be used for the separation.

The shape of the pores of the separation device may be any shape that enables passage of the adherent cells, enabling separation of the adherent cells and the modified dissolvable culture carrier. Examples of the shape of the pores of the separation device include circular, oval, rectangular and polygonal shapes.

The pore size of the separation device may be selected appropriately with consideration of the size of the adherent cells and the size of the dissolvable culture carrier and the like. For example, the pore size of the separation device is preferably larger than the size of the adherent cells, but smaller than the size of the modified dissolvable culture carrier. Specifically, the pore size of the separation device is preferably larger than the longest diameter of the adherent cells, but smaller than the shortest diameter of the modified dissolvable culture carrier. The pore size of the separation device may be, for example, within a range from 20 to 50 µm, from 20 to 40 µm, or from 20 to 30 µm.

In this disclosure, the pore size of the separation device means the pore diameter in those cases where the pore shape is circular, and in those cases where the pore shape is a shape other than circular, means the length of the longest straight line of any straight line passing through the center of the pore in a pore cross-section.

The adherent cells separated and harvested from the modified dissolvable culture carrier may be washed with a phosphate buffer solution or the like.

### [Cell Harvest method]

A cell harvest method according to one embodiment of the disclosure may include: providing a cell suspension containing a cell complex which contains adherent cells and a dissolvable culture carrier that is larger than the size of the adherent cells, and has the adherent cells disposed on the surface of the dissolvable culture carrier, subjecting the cell complex in the cell suspension to a modification treatment that modifies at least a portion of the surface of the dissolvable culture carrier in order to detach the adherent cells from the surface of the dissolvable culture carrier, and separating and harvesting the adherent cells in the cell suspension from the modified dissolvable culture carrier that is larger than the size of the adherent cells on the basis of the size difference.

By using this cell harvest method, the adherent cells disposed on the surface of the dissolvable culture carrier can be separated efficiently from the dissolvable culture carrier and harvested with high purity.

The cell harvest method may include providing a cell suspension containing a cell complex which contains adherent cells and a dissolvable culture carrier that is larger than the size of the adherent cells, and has the adherent cells disposed on the surface of the dissolvable culture carrier.

The adherent cells may be a culture of cells that have been disposed on the surface of the dissolvable culture carrier and subjected to suspension culture. In this case, the culture of cells that have been disposed on the surface of the dissolvable culture carrier and subjected to suspension culture may be procured, and the cell harvest method of the disclosure then executed, or alternatively, a typical adherent cell suspension culture may be conducted using a dissolvable culture carrier, and the cell harvest method of the disclosure then executed thereafter.

The cell suspension may contain a cell complex in which the adherent cells are disposed on the surface of the dissolvable culture carrier. The types of cells and carriers described above may be used as the adherent cells and the dissolvable culture carrier respectively. Details regarding the cell complex containing the adherent cells disposed on the surface of the dissolvable culture carrier are as described above.

In the cell suspension, the adherent cells may be disposed on part of, or across the entirety of, the surface of the dissolvable culture carrier.

Examples of the aqueous medium included in the cell suspension include liquid such as culture media, physiological saline solution, and phosphate buffers and the like.

The cell harvest method may include subjecting the cell complex in the cell suspension to a modification treatment that modifies at least a portion of the surface of the dissolvable culture carrier in order to detach the adherent cells from the surface of the dissolvable culture carrier.

The modification treatment may simply employ, as is, the modification treatment described in the culture manufacturing method of the disclosure.

The cell harvest method may include separating and harvesting the adherent cells in the cell suspension from the modified dissolvable culture carrier that is larger than the size of the adherent cells on the basis of the size difference. The method used for separating and harvesting the cells may simply employ, as is, the modification treatment described in the culture manufacturing method of the disclosure.

The adherent cells separated and harvested from the modified dissolvable culture carrier may be washed with a phosphate buffer solution or the like.

The harvested adherent cells may exist in the form of a cell suspension that is combined with a liquid such as a culture medium, physiological saline solution, or phosphate buffer solution or the like. In those cases where the cells are harvested as a cell suspension, the cell harvest method may also include subjecting the cell suspension to a cryopreservation treatment. This is able to yield a cryopreserved product of the adherent cells having very few impurities.

### [Cell-Containing Composition]

A cell-containing composition according to one embodiment of the disclosure may contain adherent cells, wherein the amount of dissolvable culture carrier residues having a size of 20 µm or smaller may be 30 particles or less per 1×10⁴ cells.

In the cell-containing composition, because the amount of dissolvable culture carrier residues having a size of 20 µm or smaller, which is similar to or smaller than the adherent cells, is 30 particles or less per 1×10⁴ cells, the adherent cells can be provided within very few impurities and high purity.

Here, the cell-containing composition may be any composition obtained following separation of the adherent cells from the dissolvable culture carrier following culturing in combination with the dissolvable culture carrier, and examples include a culture of adherent cells obtained by culturing the adherent cells in combination with the dissolvable culture carrier and then subjecting the dissolvable culture carrier to an enzyme treatment, a culture obtained using the culture manufacturing method according to one embodiment of the disclosure, a cell suspension obtained using the cell harvest method according to another embodiment of the disclosure, or a cryopreserved product of any of these compositions.

The term "dissolvable culture carrier residues" means substances obtained as a result of the treatment used for separating the dissolvable culture carrier from the adherent cells, and examples include dissolution residues (undissolved residues) of the dissolvable culture carrier obtained following a dissolution treatment, and modification residues of the dissolvable culture carrier following a modification treatment. Examples of the dissolution residues include sugar chain fragments in those cases where an enzyme is used as a surface modifier, and polypeptide fragments and the like. The cell-containing composition may contain residues from the dissolvable culture carrier modification treatment as well as the adherent cells.

The amount of dissolvable culture carrier residues, expressed as the amount of residues of 20 µm or smaller, and preferably the amount of residues of at least 1 µm but not more than 20 µm, may be not more than 30 particles, not more than 25 particles, not more than 20 particles, not more than 15 particles, not more than 10 particles, not more than 8 particles, or even 5 particles or fewer, per 1×10⁴ cells, and the amount of residues of 1 µm or greater may be 0, or may be 1 or greater.

Here, the method used for calculating the amount of dissolvable culture carrier residues having a size of 20 µm or smaller per 1×10⁴ cells in the cell-containing composition may, for example, be conducted in the following manner. A portion of the cell-containing composition is sampled, and a microscope such as a phase contrast microscope is used to observe or capture images of, for example, 5 visual fields at a magnification of 40×. At this time, a color reagent known to react with a constituent component of the dissolvable culture carrier may be used to stain the carrier. In those cases where the dissolvable culture carrier is composed of a sugar, a colorant capable of dyeing the sugar may be used. Subsequently, the number of cells, and the number of dissolvable culture carrier residues having a size of 20 µm or smaller, or a size of at least 1 µm but not more than 20 µm, observed in a prescribed region are each counted.

The cell-containing composition can be confirmed as a composition obtained from the aforementioned modification treatment of the surface of the dissolvable culture carrier using the following method. Namely, the cell suspension obtained following the modification treatment is passed through a mesh having a pore size of at least 20 µm, and the filtrate is then inspected with a phase contrast microscope at a magnification of at least 40×. In the case of a cell-containing composition that has undergone a modification treatment, at least one residue of 1 µm or smaller can be confirmed per visual field in this inspection. A method in which the obtained filtrate is collected and the existence or absence of residues is confirmed may also be used as another confirmation method. The determination of a residue or the existence of residues can be confirmed by conventional confirmation methods such as coloring reactions and tests using dyes or the like.

The culture manufacturing method according to one embodiment of the disclosure and the cell harvest method according to one embodiment of the disclosure enable a culture to be manufactured or cells to be harvested simply and with good precision, without imparting excessive load on the adherent cells. As a result, the obtained culture and adherent cells are able to exhibit a high survival rate.

The culture obtained using the culture manufacturing method according to one embodiment of the disclosure, the cells harvested using the cell harvest method according to one embodiment of the disclosure, and the cell-containing composition according to one embodiment of the disclosure contain very few impurities besides the cells, such as the dissolvable culture carrier or dissolution residues of the dissolvable culture carrier. As a result, the culture obtained using the culture manufacturing method according to one embodiment of the disclosure, the cells harvested using the cell harvest method according to one embodiment of the disclosure, and the cell-containing composition according to one embodiment of the disclosure can be used favorably as cells for a cell preparation or as a cell-containing composition.

### [Kit]

A kit according to one embodiment of the disclosure includes a dissolvable cell culture carrier, and instructions. The instructions preferably disclose the use of the dissolvable cell culture carrier in the culture manufacturing method according to one embodiment of the disclosure or the cell harvest method according to one embodiment of the disclosure. The dissolvable cell culture carrier disclosed in the instructions may be solely the dissolvable cell culture carrier disclosed in the culture manufacturing method according to one embodiment of the disclosure or the cell harvest method according to one embodiment of the disclosure, may be another dissolvable cell culture carrier, or may be a combination of these carriers.

A kit according to another embodiment of the disclosure includes a surface modifier for a modification treatment of a dissolvable cell culture carrier, and instructions. The instructions preferably disclose the use of the surface modifier in the culture manufacturing method according to one embodiment of the disclosure or the cell harvest method according to one embodiment of the disclosure. The surface modifier disclosed in the instructions may be solely the surface modifier disclosed in the culture manufacturing method according to one embodiment of the disclosure or the cell harvest method according to one embodiment of the disclosure, may be another surface modifier, or may be a combination of these surface modifiers.

A kit according to yet another embodiment of the disclosure includes a dissolvable cell culture carrier, a surface modifier for a modification treatment of the dissolvable cell culture carrier, and instructions. The instructions preferably disclose the use of the dissolvable cell culture carrier and the surface modifier in the culture manufacturing method according to one embodiment of the disclosure or the cell harvest method according to one embodiment of the disclosure. The surface modifier disclosed in the instructions may be solely the surface modifier disclosed in the culture manufacturing method according to one embodiment of the disclosure or the cell harvest method according to one embodiment of the disclosure, may be another surface modifier, or may be a combination of these surface modifiers. The dissolvable cell culture carrier disclosed in the instructions may be solely the dissolvable cell culture carrier disclosed in the culture manufacturing method according to one embodiment of the disclosure or the cell harvest method according to one embodiment of the disclosure, may be another dissolvable cell culture carrier, or may be a combination of these carriers.

In the kit according to one embodiment of the disclosure, because the dissolvable cell culture carrier, the surface modifier, or the combination of these items included in the kit is provided together with instructions describing one or a plurality of embodiments of these items, the embodiment of the dissolvable cell culture carrier, the surface modifier, or the combination thereof can be realized quickly and simply.

In each of the kits described above, details regarding the culture manufacturing method according to one embodiment of the disclosure, or the cell harvest method according to one embodiment of the disclosure are as described above, and details regarding the dissolvable cell culture carriers or surface modifiers that may be used in each method are also as described above. Each of the above kits may also include a culture medium, physiological saline solution, phosphate buffer solution, separation device such as a filter, container, or a combination of two or more of these items.

The instructions included in each of the above kits may include any medium capable of conveying the content of the instructions to a user. The instructions may be, for example, an included document having the instructional content printed on paper or some other form of sheet-like material, a packaging material such as a packaging container, packaging paper or packaging bag with the instructional content printed thereon, or a computer readable item having the instructional content recorded as data on an optical disc such as a CD, DVD or Blu-ray disc, or some other form of recording medium such as flash memory or a magnetic disc. Alternatively, the instructions may be the address of an internet site which provides the instructional content, with that internet site address printed or recorded on an included document, packaging material, or data recording medium or the like. The instructions may provide a single set of instructions per one package of the dissolvable cell culture carrier or surface modifier, or may provide one set or a plurality of sets of instructions for a plurality of packages of the dissolvable cell culture carrier or surface modifier.

### EXAMPLES

The disclosure is described below in further detail using a series of examples, but provided the technical scope of the disclosure is not exceeded, the disclosure is not limited to the following examples.

### [Examples 1 to 3]

### (1) Cell Culture

Cells were cultured using a 6-well plate. Specifically, human mesenchymal stem cells (MSCs) were cultured in wells having a volume of 2.0 mL/well. A medium prepared by adding 10% (v/v) of FBS (fetal bovine serum) to 1.0 L of αMEM was used as the culture medium. A microcarrier that functioned as a culture carrier was added to the culture medium together with the cells, and a shaking culture was conducted in a CO₂ incubator maintained at a temperature of 37°C and a CO₂ concentration of 5%. Following start of the culture, a cell complex having the cells disposed on the surface of the microcarrier was observed.

Cytodex 1 (a crosslinked dextran having N,N-diethylaminoethyl groups, D50: 180 µm) (Cytiva Co., Ltd.) was used as the microcarrier. Confirmation of the minimum diameter of Cytodex 1 revealed a value exceeding 50 µm.

The cell seeding density was 3,000 cells/mL, and the initial addition amount of the microcarrier was 0.001 g/mL (5cm²/mL).

### (2) Cell harvest

Following a one-week culture, the cell suspension containing the cells and the microcarrier in the form of a cell complex was harvested from the 6-well plate containing the cells, and was washed twice with DPBS (Dulbecco's phosphate buffer solution). Following washing, the supernatant was aspirated, a 37°C reagent prepared with a dextranase (Sigma-Aldrich Corporation) and a trypsin-EDTA solution (0.25 w/v%, 0.02 w/v%, Sigma-Aldrich Corporation) was added to each well in an amount of 1 mL/well, and the plate was then left to stand. The final dextranase concentration, relative to the total volume of the cell suspension in each well, was set to 0.1 v/v%, 0.01 v/v% and 0.001 v/v% respectively, and the final trypsin concentration was set to 0.2 w/v%. The pH of the culture medium at this time was within a range from 7.1 to 7.2. After 10 minutes had elapsed, the cells were observed with a phase contrast microscope (observation result 1).

Each of the modified cell suspensions was pipetted 10 times, and then observed again with a phase contrast microscope (observation result 2). Subsequently, the cell suspension was passed through a mesh (cell strainer, Corning Inc.) having a pore size of 20 µm, the cells were harvested, and the cell harvest rate and cell survival rate were evaluated using a NucleoCounter (a registered trademark) NC-200, ChemoMetec A/S. The results are shown in Table 1.

The cell harvest rate is the percentage of the number of cells harvested relative to the number of cells following the culture. The cell survival rate is the percentage of the number of live cells relative to the number of cells harvested.

### [Comparative Example 1]

In step (2) of Example 1, following washing of the cell suspension and subsequent aspiration of the supernatant, with the exception of adding a reagent prepared with only the 37°C trypsin-EDTA solution (0.25 w/v%, 0.02 w/v%) (final trypsin concentration: 0.25 w/v%) to each well in an amount of 1 mL/well, treatment was conducted in the same manner as Example 1. Subsequently, the cells were harvested and the cell harvest rate and cell survival rate were evaluated in the same manner as Example 1. The results are shown in Table 1.

### [Comparative Example 2]

In step (2) of Example 1, following harvest of the cells and washing with DPBS, dextranase 500 U/mg (product number: D0443-250ML, Sigma-Aldrich Corporation) was added to each well in accordance with the product protocol for the microcarrier, in an amount sufficient to achieve a concentration of 10 mg/50mL per 50 mL of the microcarrier, and a microcarrier dissolution treatment was then conducted at 37°C and a pH of 6.0 for 20 minutes. Subsequently, the cells were harvested and the cell harvest rate and cell survival rate were evaluated in the same manner as Example 1. The results are shown in Table 1.

### [Comparative Example 3]

In step (2) of Example 1, with the exceptions of altering the dextranase concentration to 1% (v/v) and extending the reaction time to 20 minutes, treatment was conducted in the same manner as Example 1. Subsequently, the cells were harvested and the cell harvest rate and cell survival rate were evaluated in the same manner as Example 1. The results are shown in Table 1.

Following the modification treatment, the cell suspension was observed under a phase contrast microscope (CKX53, Olympus Corporation), and the observation result (the aforementioned observation result 1) was evaluated against the following criteria. The results are shown in Table 1.

A: both the microcarrier and the cells were observed, but microcarriers having a size smaller than the cells were not more than 30 per 1×10⁴ cells, or were not observed.

B: the cells were observed, but the particulate microcarrier had dissolved and was not observed, although a plurality of residues derived from the microcarrier and having a size similar to or smaller than the cells were observed.

**[Table 1]**

| | Modification treatment | Evaluation of residues observed in modified cell suspension | After cell harvest | |
|---|---|---|---|---|
| | | | Cell harvest rate (%) | Cell survival rate (%) |
| Example 1 | 0.1% Dextranase / Trypsin | A | 17.2 | 91.8 |
| Example 2 | 0.01% Dextranase / Trypsin | A | 34.5 | 96.5 |
| Example 3 | 0.001% Dextranase / Trypsin | A | 87.0 | 96.0 |
| Comparative Example 1 | Trypsin | A | 8.1 | 95.5 |
| Comparative Example 2 | Dextranase 500 U/mg | B | about 100 | 95.5 |
| Comparative Example 3 | 1% Dextranase / Trypsin | B | 95 | 95 |

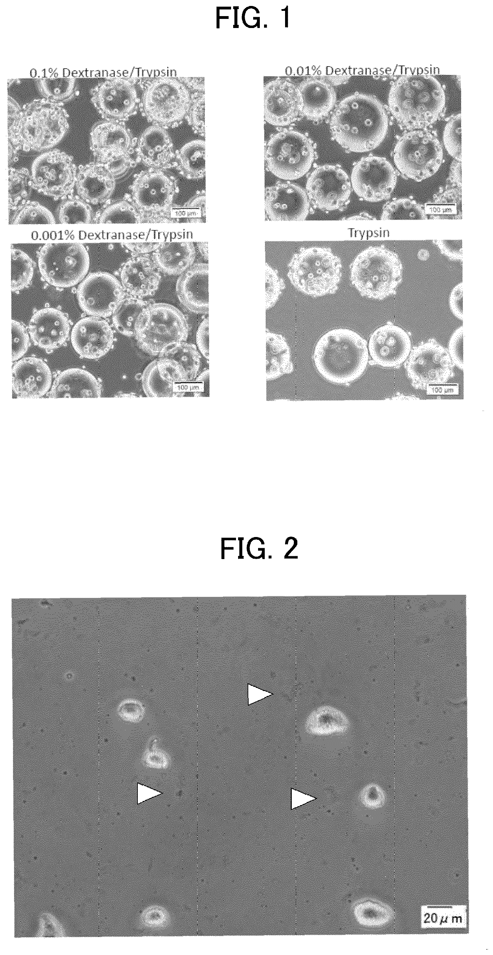

FIG. 1 and FIG. 2 show a series of photographic images obtained by observing the cell suspensions following modification treatment with a phase contrast microscope in the following examples and comparative examples.

FIG. 1 are photographic images of the above observation result 1 at a magnification of 10×, wherein the upper left image in FIG. 1 shows Example 1, the upper right image in FIG. 1 shows Example 2, the lower left image in FIG. 1 shows Example 3, and the lower right image in FIG. 1 shows Comparative Example 1. FIG. 2 is a photographic image of the above observation result 2 at a magnification of 40×, and shows Comparative Example 3.

In Examples 1 to 3, as shown in Table 1, the cell harvest rates were all at least 15%, and the cell survival rates were all 90% or higher. Further, as shown in FIG. 1, when the cell suspension was observed following the modification treatment, both the cells and the incompletely dissolved microcarriers were observed. When observation was conducted with the phase contrast microscope, and the measure attached to the microscope was used to confirm the minimum diameter of the microcarrier across 5 visual fields, the value exceeds 50 µm in each example, and when the average of the minimum diameter was taken and compared with the size of the microcarrier prior to the modification treatment, the resulting value was 90% or higher in each case. On the other hand, when the same method was used to attempt to confirm the existence of residues such as dissolution residues of the microcarrier, residues such as microcarrier dissolution residues with a size similar to or smaller than the size of the cells could not be confirmed (see FIG. 1). As a result, by using a mesh to harvest the cells, the microcarrier was able to be easily separated, enabling only the cells to be harvested with good precision.

In contrast, in Comparative Example 1, as shown in Table 1, the cell harvest rate was low, with the rate falling below 10%. Further, as shown in Table 1, when the cell suspension was observed following the modification treatment, both the cells and the incompletely dissolved microcarriers were observed in the cell suspension. When the number of residues was confirmed using the same method as described above, residues such as microcarrier dissolution residues with a size similar to or smaller than the size of the cells could not be confirmed. As a result, by using a mesh to harvest the cells, the cells were able to be easily separated from the microcarrier, but the cells tended to remain stuck to the surface of the microcarriers retained on the mesh, with many adherent cells still present. Based on these results, it was evident that satisfactory detachment of the cells from the microcarrier could not be achieved with only a cell detachment agent.

In Comparative Example 2 and Comparative Example 3, when the cell suspension was observed following the modification treatment, the microcarrier had almost completely dissolved, and instead, many cell aggregates formed from a plurality of cells were observed. When the cell suspension was observed following pipetting, the cell aggregates that had been confirmed prior to the pipetting had all been dispersed as single cells, but as well as the cells, residues of the microcarrier existed in large amounts in the form of fine particles with a size of 20 µm or smaller, in other words, fine particles having a size smaller than the cells (see FIG. 2 for Comparative Example 3, a similar result was observed for Comparative Example 2, but this result is not shown in the drawings). The arrows illustrated at three locations in FIG. 2 indicate residues of a size smaller than the cells, namely dissolution residues.

Furthermore, in Comparative Example 2 and Comparative Example 3, as shown in Table 1, the cell harvest rates and cell survival rates were high, with those high cell harvest rates being due to the dissolution of the entire microcarrier.

However, as mentioned above, each of the cell suspensions contained a large amount of microcarrier residues with a size of 20 µm or smaller, and even when an attempt was made to harvest only the cells with a mesh or the like, the cells were contaminated with a large amount of residues, and separating only the cells with a high level of precision was impossible.

The disclosure is related to the subject matter disclosed in prior Japanese Application 2021-060494 filed on March 31, 2021, the entire content of which is incorporated by reference herein. It should be noted that, in addition to the embodiments already described, various modifications and alterations may be made to these embodiments without departing from the novel and advantageous features of the disclosure. Accordingly, it is intended that all such modifications and alterations are included within the scope of the appended claims.

## Claims

1. A culture manufacturing method comprising:
bringing adherent cells into contact with a dissolvable culture carrier having a size larger than that of the adherent cells, and disposing the adherent cells on a surface of the dissolvable culture carrier,
subjecting the adherent cells disposed on the surface of the dissolvable culture carrier to suspension culture in a culture medium,
subjecting the dissolvable culture carrier to a modification treatment that modifies at least a portion of the surface in order to detach the adherent cells in the suspension culture from the surface of the dissolvable culture carrier, and
following the modification treatment, separating and harvesting the adherent cells from the modified dissolvable culture carrier having a size larger than that of the adherent cells based on size difference.

2. The culture manufacturing method according to Claim 1, wherein the dissolvable culture carrier comprises a polysaccharide, a protein, or a combination thereof.

3. The culture manufacturing method according to Claim 1 or 2, wherein the dissolvable culture carrier comprises at least one substance selected from the group consisting of dextran, cellulose, collagen, gelatin, polygalacturonic acid, alginic acid, and derivatives thereof.

4. The culture manufacturing method according to any one of Claims 1 to 3, wherein the dissolvable culture carrier is porous.

5. The culture manufacturing method according to any one of Claims 1 to 4, wherein the modification treatment comprises modifying the dissolvable culture carrier using a surface modifier.

6. The culture manufacturing method according to any one of Claims 1 to 5, wherein the adherent cells and the modified dissolvable culture carrier are separated using a separation device.

7. The culture manufacturing method according to Claim 6, wherein the separation device has a pore size of 20 to 50 µm.

8. The culture manufacturing method according to any one of Claims 1 to 7, wherein a size of the modified dissolvable culture carrier has a minimum diameter that exceeds 50 µm.

9. A cell harvest method comprising:
providing a cell suspension containing a cell complex, which comprises adherent cells and a dissolvable culture carrier having a size larger than that of the adherent cells, and has the adherent cells disposed on a surface of the dissolvable culture carrier,
subjecting the cell complex in the cell suspension to a modification treatment that modifies at least a portion of the surface of the dissolvable culture carrier in order to detach the adherent cells from the surface of the dissolvable culture carrier, and
separating and harvesting the adherent cells in the cell suspension from the modified dissolvable culture carrier having a size larger than that of the adherent cells based on size difference.

10. A kit comprising a dissolvable cell culture carrier, and instructions that disclose use of the dissolvable cell culture carrier in the culture manufacturing method according to any one of Claims 1 to 8 or the cell harvest method according to Claim 9.

11. A kit comprising a surface modifier for a modification treatment of a dissolvable cell culture carrier, and instructions that disclose use of the surface modifier in the culture manufacturing method according to any one of Claims 1 to 8 or the cell harvest method according to Claim 9.

12. A cell-containing composition comprising adherent cells, wherein an amount of dissolvable culture carrier dissolution residues having a size of 20 µm or smaller is 30 particles or less per 1×10⁴ cells.
